(19) Europäisches Patentamt
European Patent Office
Office européen des brevets

(11) Publication number: **0 110 536 B1**

(12) **EUROPEAN PATENT SPECIFICATION**

(45) Date of publication of patent specification: **21.09.88**

(21) Application number: **83306310.0**

(22) Date of filing: **18.10.83**

(51) Int. Cl.[4]: **C 07 C 25/24,** C 07 C 17/26, C 07 D 249/08

(54) **Substituted alkenes and processes for their preparation.**

(30) Priority: **10.11.82 GB 8232083**
**01.02.83 GB 8302751**

(43) Date of publication of application:
**13.06.84 Bulletin 84/24**

(45) Publication of the grant of the patent:
**21.09.88 Bulletin 88/38**

(84) Designated Contracting States:
**BE CH DE FR GB IT LI NL**

(56) References cited:
**EP-A-0 017 852**
**EP-A-0 047 594**
**EP-A-0 093 526**
**AU-B-2 765 977**
**GB-A-2 064 520**

**JOURNAL OF ORGANIC CHEMISTRY, vol. 47, october 22, 1982, EASTON (US); J.M. HORNBACK et al.: "Photochemistry of (o-Methylphenyl)alkenes and the Stereospecific Trapping of the Resulting O-Xylylenes", pages 4285-4291.**

(73) Proprietor: **IMPERIAL CHEMICAL INDUSTRIES PLC**
**Imperial Chemical House Millbank**
**London SW1P 3JF (GB)**

(72) Inventor: **Jones, Raymond Vincent Heavon**
**26 Clarendon Road**
**Linlithgow West Lothian Scotland (GB)**
Inventor: **Fleming, Ian George Cameron**
**56 Newmains Road**
**Kirkliston Scotland (GB)**
Inventor: **Ewins, Robert Comgall**
**23 Tantallon Drive Carron**
**Falkirk Stirlingshire FK2 8DJ Scotland (GB)**
Inventor: **Jones, John David**
**57 Keats Road**
**Greenmount Bury BL8 4EP (GB)**

(74) Representative: **Houghton, Malcolm John et al**
**Imperial Chemical Industries PLC**
**Legal Department: Patents**
**PO Box 6 Welwyn Garden City**
**Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 110 536 B1

Courier Press, Leamington Spa, England.

(56) References cited:
JOURNAL OF CHEMICAL RESEARCH SYNOPSES", 1977, no. 4; G. CASIRAGHI et al.: "Regiospecificity in Reactions of Phenoxymagnesium Bromides with Ketals: One-Step ortho-Alkenylation of Phenols", page 96.

JOURNAL OF ORGANIC CHEMISTRY, vol. 42, August 5, 1977, EASTON (US); T. KAMETANI et al.: "Competitive Reactions between Sigmatropic Reaction and Cycloaddition Affected by the Geometry of o-Quinodimethanes", pages 2672-2676.

CHEMICAL ABSTRACTS, vol. 79, no. 13, October 1, 1973, page 450, abstract 78242f, COLUMBUS, OHIO (US); Y.S. SHABAROV et al.: "Bromination and acetylation of 1-methyl-1- Phenylcyclopropane".

CHEMICAL ABSTRACTS, vol. 78, no. 11, march 19, 1973, page 425, abstract 71588u,

COLUMBUS, OHIO (US); Y.U. SHABAROV et al.: "1-Methyl-1-(o-nitrophenyl) cyclopropane and its reaction with concentrated sulfuric acid".

TETRAHEDRON, vol. 25, 1969, Pergamon press, GB; K.M. BAKER et al.: "Hydrogen abstraction from methyl groups attached to an aromatic ring", pages 2487-2492.

CHEMICAL ABSTRACTS, vol. 70, no. 15, april 14, 1969, page 317, abstract 67820f,

COLUMBUS, OHIO (US); K.G. TASHCHUK et al.: "Application of phosphoryl-olefination for synthesis of some esters of beta-alkyl-beta-arylacrylic acids and obtaining substituted styrenes".

THE JOURNAL OF ORGANIC CHEMISTRY, vol. 34, April 1969, EASTON (US); H. TANIDA et al.: "Rates and Products of Acetolysis of p-Nitroneophyl p-Bromobenzenesulfonate", pages 1086-1089

CHEMICAL ABSTRACTS, 8th Collective Index, compound 10, 1967-1971, Subjects Amino-Benzimidazolid, American Chemical Society, COLUMBUS, OHIO (US)

CHEMICAL ABSTRACTS, Registry Handbook, number section 1965-1971, Registry numbers 4600-00-4 through 12699-98-8, American Chemical Society, COLUMBUS, OHIO (US)

THE JOURNAL OF ORGANIC CHEMISTRY, vol. 33, July 1968, Easton (US); D.J. PASTO et al.: "Reaction of w-Chloro Ketones with Methylene-triphenylphosphorane", pages 2975-2977.

(56) References cited:
Ullmanns Enzyklopädie der techn. Chemie, 4. Auflage, vol. 10, 1975, p. 564

**Description**

This invention relates to certain substituted alkenes, processes for their preparation and their use in the synthesis of fungicidal compounds.

According to one aspect of the present invention there are provided substituted alkenes of formula (II):

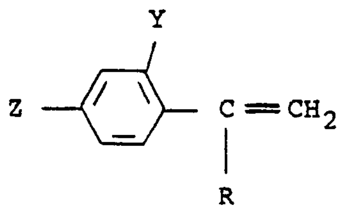

(II)

in which Y and Z are independently hydrogen, chloro or fluoro, provided at least one of Y and Z is chlor or fluoro, and R is $C_3$ or $C_4$ alkyl.

An example of a preferred substituted alkene is 2-(2,4-dichlorophenyl)-*n*-hex-1-ene.

According to another aspect of the invention there is provided a process for the preparation of the compounds of formula (II) which comprises reacting a compound of formula (III):

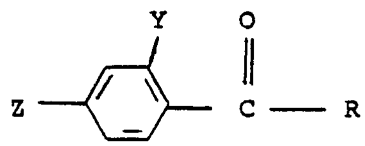

(III)

in which R, Y and Z have the meanings stated above, with methylenetriphenylphosphorane.

Methylene-triphenylphosphorane is a known reagent for use in the Wittig reaction i.e. the reaction between an alkylidenephosphorane and a carbonyl compound to form a compound containing a

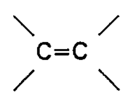

bond. A method for its preparation is described in the Journal of Organic Chemistry, 1963, *28*, p. 1128 and outlined by the following reaction scheme.

Reaction scheme for the preparation of methylene-triphenyl phosphorane

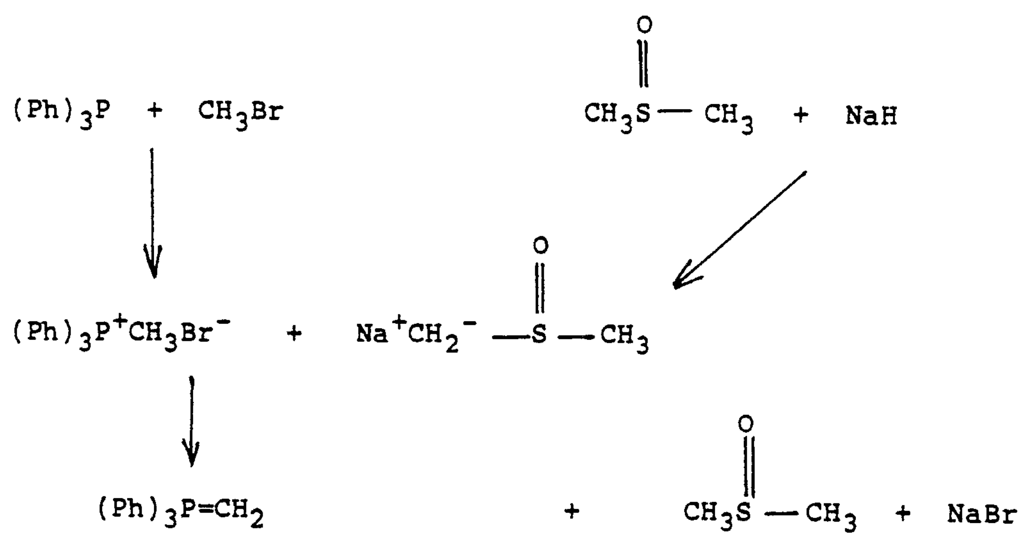

methylene-triphenylphosphorane

[Ph is phenyl]

Preferably the compound of formula (III) is added as a solution in dimethylsulphoxide to the methylene-triphenylphosphorane which, itself, is obtained by the above method as a solution in dimethyl sulphoxide.

The compound of formula (III) may be obtained by Friedel-Crafts reaction between an acyl chloride of formula RCOCl and a benzene derivative of formula (IV):

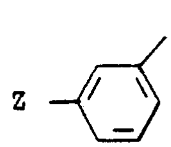

(IV)

in which R, Y and Z have the meanings stated above, in the presence of a Lewis acid such as aluminium chloride.

The substituted alkenes of formula (II) are useful intermediates for fungicides of the formula (VII):

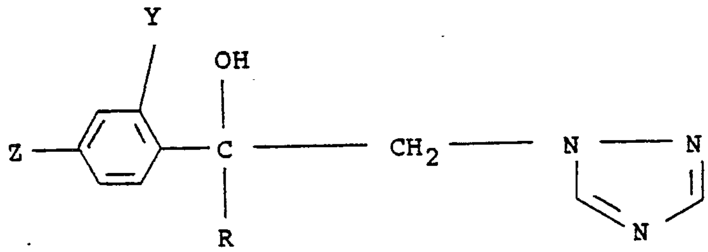

(VII)

in which R, Y and Z have the meanings stated above, which are described in, for example, European Patent Specification No. 48548 (European Application No. 81303753.8) and UK Patent Specification No. 2064520.

Thus the present invention also includes the use of the substituted alkenes in the synthesis of the fungicidal compounds of formula (VII) by a process which comprises the steps of

(i) reacting an alkene of formula (I) with chlorine, bromine, hypochlorous acid or hypobromous acid in aqueous medium to give the correspondiong halohydrin of formula (VIII):

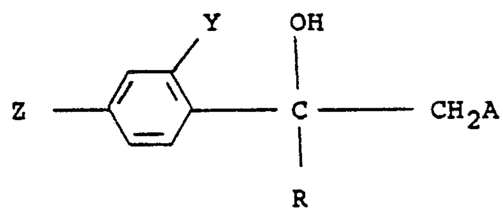

(VIII)

in which A is chlorine or bromine and R, Y and Z have the meanings previously stated; and

(ii) reacting the halohydrin so formed with 1,2,4-triazole in the presence of an acid binding agent or with an alkali metal salt of the triazole, conveniently in a solvent such as dimethylformamide.

Step (ii) is thought to proceed via the epoxide of formula (IX):

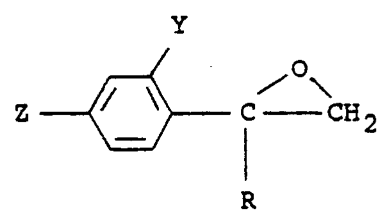

(IX)

which is readily formed from the halohydrin under the basic conditions of the triazole addition.

Alternatively, the fungicidal compounds of formula (VII] are prepared by a process which comprises the steps of

(a) forming directly the epoxide of formula (IX) by the electrochemical epoxidation of the alkene of formula (I) by a process such as that described in European Application No. 83302141.3 (Publication No. 0093526) which comprises electrolysing the alkene in a medium comprising a water-miscible solvent and aqueous solution of a halogen salt; and

(b) reacting the epoxide so formed with 1,2,4-triazole in the presence of an acid binding agent or with an alkali metal salt of the triazole conveniently in a solvent such as dimethylformamide.

The invention is illustrated by the following Example in which parts and percentages are by weight.

Example

(a) 2-(2,4-Dichlorophenyl)-n-hex-1-ene

A solution of 2,4-dichlorovalerophenone (18.4 parts) in dimethylsulphoxide (22 parts) was added to the solution of methylenetriphenylphosphorane (27.6 parts) prepared as described in J. Org. Chem. 1963, *28*,

p.1128. After stirring overnight at ambient temperature, the amber solution was heated to 70°C for one hour, and then cooled to 10°C. The solution was drowned into ice water (500 parts), stirred for 30 minutes, and filtered. The filter cake was washed with hexane (132 parts) in four lots, and this was also used to wash the filtrate. The hexane was dried and evaporated to yield 15 parts of an oil, which was shown by NMR analysis to contain 11.8 parts of pure 2-(2,4-dichlorophenyl)-hex-1-ene (64.1% yield). This boiled at 98—103°C/3 mm Hg (Analysis C 63.2, H 6.3, Cl 30.6. $C_{12}H_{14}Cl_2$ requires C 62.88, H 6.16, Cl 30.96).

(b) 1-(1,2,4-Triazol-1-yl)-2-(2,4-dichlorophenyl)-hexan-2-ol

2-(2,4-Dichlorophenyl)-hex-1-ene (6 parts) was suspended in a mixture of water (7 parts) and N,N-dimethylformamide (12.9 parts) at 30—35°C, and was brominated at this temperature with bromine (5.0 parts) and 100 volume hydrogen peroxide (3.2 parts). The mixture settled into two layers, the lower layer containing a mixture of 1-bromo-2-(2,4-dichlorophenyl)-hexan-2-ol and 1,2-dibromo-2-(2,4-dichlorophenyl)-hexane in aqueous dimethylformamide.

The lower layer (7.9 parts) was heated at 80—85°C in dimethylformamide (16.7 parts) with 1,2,4-triazole (1.7 parts) and potassium carbonate (8.7 parts) until NMR spectroscopy showed no residual 1-bromo-2-(2,4-dichlorophenyl)-hexan-2-ol. The mixture was drowned into water, and the organic layer was extracted with chloroform (4 × 75 parts). Evaporation of the solvent yielded an oil containing 1.7 parts (20.5% yield) of 1-(2,3,4-triazol-1-yl)-2-(2,4-dichlorophenyl)-hexan-2-ol, identified by P.M.R. Spectroscopy and H.P.L.C. analysis.

**Claims**

1. Substituted alkenes of formula (II):

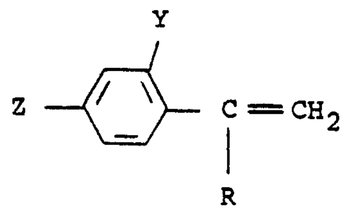

(II)

in which Y and Z are independently hydrogen, chloro or fluoro provided at least one of Y and Z is chloro or fluoro and R is $C_3$ or $C_4$ alkyl.

2. 2-(2,4-dichlorophenyl)-*n*-hex-1-ene.

3. Process for the preparation of substituted alkenes according to claim 1 which comprises reacting a compound of formula (III)

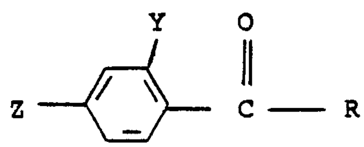

(III)

in which R, Y and Z have the meaning given in claim 1, with methylene triphenylphosphorane.

4. Use of substituted alkenes according to claim 1 in the synthesis of fungicidal compounds of formula (VII):

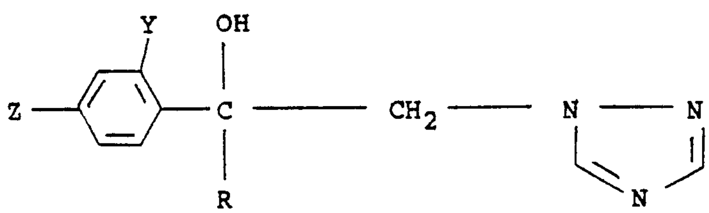

(VII)

by a process which comprises the steps of (i) reacting an alkene of formula (II) with chlorine, bromine, hypochlorous acid or hypobromous acid in aqueous medium to give the corresponding halohydrin of formula (VIII):

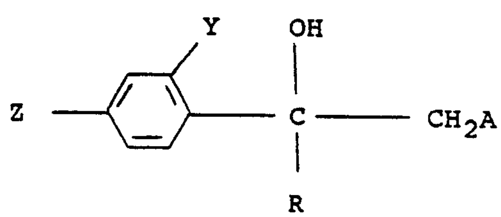

(VIII)

in which A is chlorine or bromine and R, Y and Z have the meanings given in claim 1; and (ii) reacting the

halohydrin so formed with 1,2,4-triazole in the presence of an acid binding agent or with an alkali metal salt of the triazole.

5. Use of substituted alkenes according to claim 1 in the synthesis of fungicidal compounds of general formula (VII):

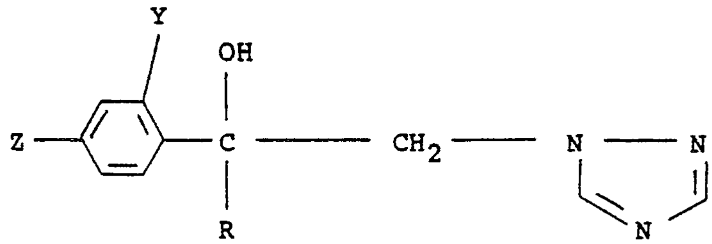

(VII)

by a process which comprises the steps of (a) epoxidising the alkene of formula (II) to form the epoxide of general formula (IX):

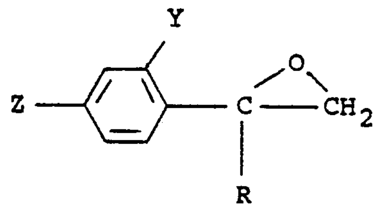

(IX)

in which R, Y and Z have the meanings given in claim 1
and
(b) reacting the epoxide so formed with 1,2,4-triazole in the presence of an acid binding agent or with an alkali metal salt of the triazole.

**Patentansprüche**

1. Substituierte Alkene der Formel (II)

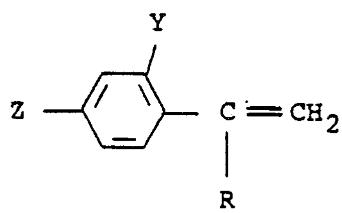

(II)

worin Y und Z unabhängig voneinander für Wasserstoff, Chloro oder Fluoro stehen, mit der Maßgabe, daß mindestens eines der Symbole Y und Z für Chloro oder Fluoro steht, und R für $C_{3-4}$-Alkyl steht.

2. 2-(2,4-Dichlorophenyl)-n-hex-1-en.

3. Verfahren zur Herstellung von substituierten Alkenen nach Anspruch 1, bei welchem eine Verbindung der Formel (III)

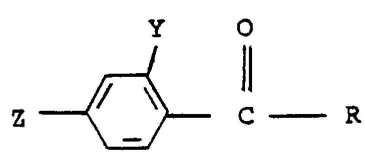

(III)

worin R, Y und Z die in Anspruch 1 angegebenen Bedeutungen besitzen, mit Methylentriphenylphosphoran umgesetzt wird.

4. Die Verwendung von unsubstituierte Alkenen nach Anspruch 1 bei der Synthese von fungiziden Verbindungen der Formel (VII)

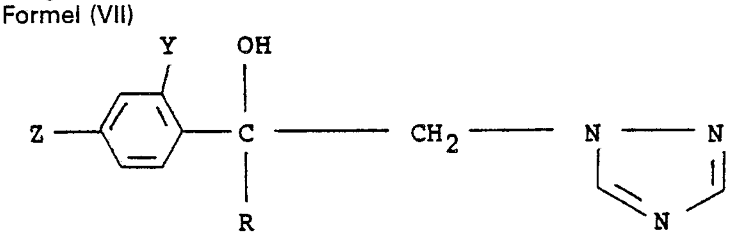

(VII)

durch ein Verfahren, bei welchem (i) ein Alken der Formel (II) mit Chlor, Brom, unterchloriger Säure oder

unterbromiger Säure in einem wäßrigen Medium umgesetzt wird, um ein entsprechendes Halogenhydrin der Formel (VIII)

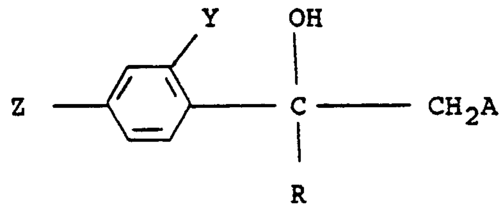

(VIII)

worin A für Chlor oder Brom steht und R, Y und Z die in Anspruch 1 angegebenen Bedeutungen besitzen, herzustellen; und (ii) das so gebildete Halogenhydrin mit 1,2,4-Triazol in Gegenwart eines säurebindenden Mittels oder aber mit einem Alkalimetallsalz dieses Triazols umgesetzt wird.

5. Die Verwendung von substituierten Alkenen nach Anspruch 1 bei der Synthese von fungiziden Verbindungen der allgemeinen Formel (VII)

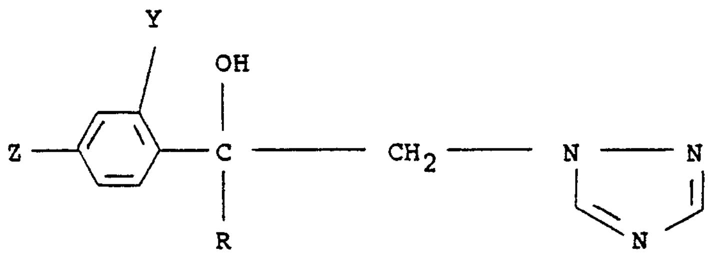

(VII)

durch ein Verfahren, bei welchem

(a) das Alken der Formel (II) oxidiert wird, um ein Epoxid der allgemeinen Formel (IX)

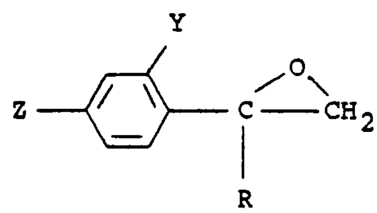

(IX)

worin R, Y und Z die in Anspruch 1 angegebenen Bedeutungen besitzen, herzustellen; und

(b) das so gebildete Epoxid mit 1,2,4-Triazol in Gegenwart eines säurebindenen Mittels oder aber mit einem Alkalimetallsalz dieses Triazols umgesetzt wird.

**Revendications**

1. Alcènes substitués répondant à la formule (II):

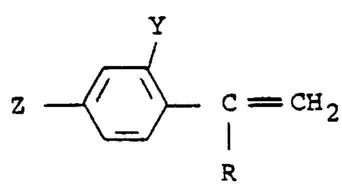

(II)

dans laquelle Y et Z représentent indépendamment l'hydrogène, un substituant chloro ou fluoro, sous réserve qu'au moins l'un de Y et Z représente un substituant chloro ou fluoro, et R est un groupe alkyle en $C_3$ ou $C_4$.

2. 2-(2,4-dichlorophényl)-*n*-hex-1-ène.

3. Procédé de préparation d'alcènes substitués suivant la revendication 1, qui consiste à faire réagir un composé répondant à la formule (III):

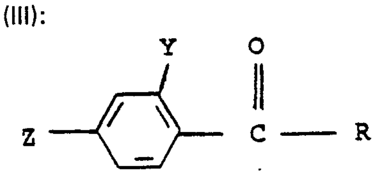

(III)

dans laquelle R, Y et Z répondent aux définitions suivant la revendication 1, avec du méthylène-triphényl-phosphorane.

4. Utilisation d'alcénes substitués suivant la revendication 1 dans la synthèse de composés fongicides répondant à la formule (VII):

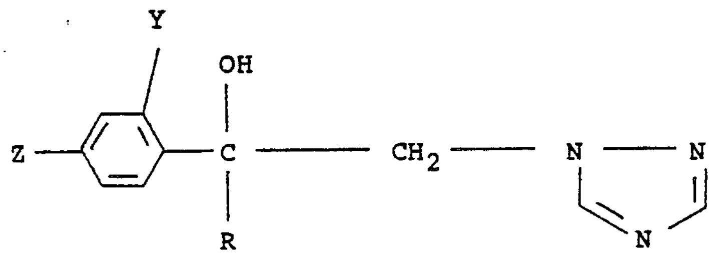

(VII)

par un procédé qui comprend les étapes consistant (i) à faire réagir un alcène de formule (II) avec le chlore, le brome, l'acide hypochloreux ou l'acide hypobromeux en milieu aqueux pour donner l'halogènhydrine correspondante répondante à la formule (VIII)

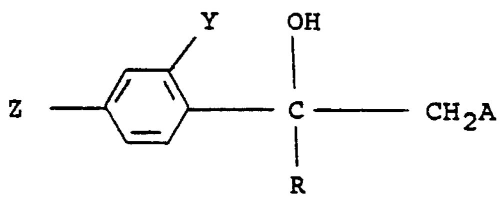

(VIII)

dans laquelle A représente le chlore ou le brome et R, Y et Z répondent aux définitions suivant la revendication 1; et (ii) à faire réagir l'halogènhydrine ainsi formée avec le 1,2,4-triazole en présence d'un agent accepteur d'acide ou bien avec un sel de métal alcalin du triazole.

5. Utilisation d'alcénes substitués suivant la revendication 1 dans la synthèse de composés fongicides répondant à la formule générale (VII):

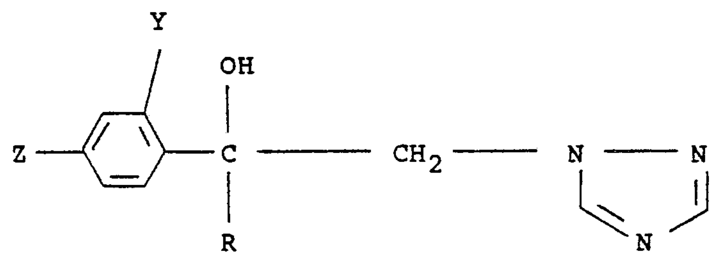

(VII)

par un procédé qui comprend les étapes consistant

(a) à effectuer l'époxydation de l'alcène de formule (II) pour former l'époxyde répondant à la formule générale (IX):

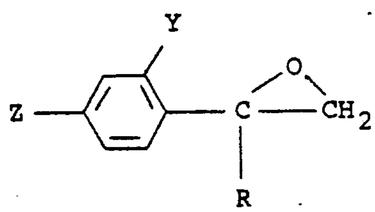

(IX)

dans laquelle R, Y et Z répondent aux définitions suivant la revendication 1, et

(b) à faire réagir l'époxyde ainsi formé avec le 1,2,4-triazole en présence d'un agent accepteur d'acide ou bien avec un sel de métal alcalin du triazole.